# NEW EUROPEAN PATENT SPECIFICATION

(11) **EP 2 131 806 B2**
(45) Date of publication and mention of the opposition decision: **18.05.2022**
(45) Mention of the grant of the patent: 05.12.2012
(21) Application number: 08735787.7
(22) Date of filing: 03.04.2008
(51) Int. Cl.: A61K 8/19, A61K 8/21, A61K 8/27, A61Q 11/00

(54) **ORAL CARE COMPOSITION COMPRISING PARTICULATE ZINC OXIDE**
MUNDPFLEGEZUSAMMENSETZUNG ENTHALTEND TEILCHENFÖRMIGES ZINKOXID
NOUVELLE UTILISATION D'UNE COMPOSITION DE SOIN DENTAIRE

(30) Priority: 05.04.2007 GB 0706787
(43) Date of publication of application: 16.12.2009
(73) Proprietor: Glaxo Group Limited, Brentford, Middlesex TW8 9GS (GB)
(72) Inventor: FOWLER, Christabel, Surrey KT13 0DE (GB)
(74) Representative: GSK Consumer Healthcare
(86) International application number: PCT/EP2008/054045
(87) International publication number: WO 2008/122578

(56) References cited:
- EP-A- 0 346 957
- WO-A-94/14406
- WO-A-94/26245
- WO-A-99/59539
- US-A- 4 863 722
- US-A- 5 455 023
- US-A1- 2007 020 201

## Description

### FIELD OF THE INVENTION

The present invention relates to the use of an oral care composition comprising particulate nano zinc oxide, and a dispersing agent in the absence of palatinit optionally together with a source of fluoride ions, for strengthening dental enamel of teeth thereby providing protection from acidic challenges. Such compositions are of use in combating (i.e. helping to prevent, inhibit and/or treat) dental erosion and/or tooth wear.

### BACKGROUND OF THE INVENTION

Tooth mineral is composed predominantly of calcium hydroxyapatite, Ca₁₀(PO₄)₆(OH)₂, which may be partially substituted with anions such as carbonate or fluoride, and cations such as zinc or magnesium. Tooth mineral may also contain non-apatitic mineral phases such as octacalcium phosphate and calcium carbonate.

Tooth loss may occur as a result of dental caries, which is a multifactorial disease where bacterial acids such as lactic acid produce sub-surface demineralisation that does not fully remineralise, resulting in progressive tissue loss and eventually cavity formation. The presence of a plaque biofilm is a prerequisite for dental caries, and acidogenic bacteria such as *Streptococcus mutans* may become pathogenic when levels of easily fermentable carbohydrate, such as sucrose, are elevated for extended periods of time.

Even in the absence of disease, loss of dental hard tissues can occur as a result of acid erosion and/or physical tooth wear; these processes are believed to act synergistically. Exposure of the dental hard tissues to acid causes demineralisation, resulting in surface softening and a decrease in mineral density. Under normal physiological conditions, demineralised tissues self-repair through the remineralising effects of saliva. Saliva is supersaturated with respect to calcium and phosphate, and in healthy individuals saliva secretion serves to wash out the acid challenge, and raises the pH so as to alter the equilibrium in favour of mineral deposition.

Dental erosion (i.e. acid erosion or acid wear) is a surface phenomenon that involves demineralisation, and ultimately complete dissolution of the tooth surface by acids that are not of bacterial origin. Most commonly the acid will be of dietary origin, such as citric acid from fruit or carbonated drinks, phosphoric acid from cola drinks and acetic acid such as from vinaigrette. Dental erosion may also be caused by repeated contact with hydrochloric acid (HCl) produced in the stomach, which may enter the oral cavity through an involuntary response such as gastroesophageal reflux, or through an induced response as may be encountered in sufferers of bulimia.

Tooth wear (ie physical tooth wear) is caused by attrition and/or abrasion. Attrition occurs when tooth surfaces rub against each other, a form of two-body wear. An often dramatic example is that observed in subjects with bruxism, a grinding habit where the applied forces are high, and is characterised by accelerated wear, particularly on the occlusal surfaces. Abrasion typically occurs as a result of three-body wear and the most common example is that associated with brushing with a toothpaste. In the case of fully mineralised enamel, levels of wear caused by commercially available toothpastes are minimal and of little or no clinical consequence. However, if enamel has been demineralised and softened by exposure to an erosive challenge, the enamel becomes more susceptible to tooth wear. Dentine is much softer than enamel and consequently is more susceptible to wear. Subjects with exposed dentine should avoid the use of highly abrasive toothpastes, such as those based on alumina. Again, softening of dentine by an erosive challenge will increase susceptibility of the tissue to wear.

Dentine is a vital tissue that in vivo is normally covered by enamel or cementum depending on the location i.e. crown versus root respectively. Dentine has a much higher organic content than enamel and its structure is characterised by the presence of fluid-filled tubules that run from the surface of the dentine-enamel or dentine-cementum junction to the odontoblast/pulp interface. It is widely accepted that the origins of dentine hypersensitivity relate to changes in fluid flow in exposed tubules, (the hydrodynamic theory), that result in stimulation of mechanoreceptors thought to be located close to the odontoblast/pulp interface. Not all exposed dentine is sensitive since it is generally covered with a smear layer; an occlusive mixture comprised predominantly of mineral and proteins derived from dentine itself, but also containing organic components from saliva. Over time, the lumen of the tubule may become progressively occluded with mineralised tissue. The formation of reparative dentine in response to trauma or chemical irritation of the pulp is also well documented. Nonetheless, an erosive challenge can remove the smear layer and tubule "plugs" causing outward dentinal fluid flow, making the dentine much more susceptible to external stimuli such as hot, cold and pressure. As previously indicated, an erosive challenge can also make the dentine surface much more susceptible to wear. In addition, dentine hypersensitivity worsens as the diameter of the exposed tubules increases, and since the tubule diameter increases as one proceeds in the direction of the odontoblast/pulp interface, progressive dentine wear can result in an increase in hypersensitivity, especially in cases where dentine wear is rapid.

Loss of the protective enamel layer through erosion and/or acid-mediated wear will expose the underlying dentine, and are therefore primary aetiological factors in the development of dentine hypersensitivity.

It has been claimed that an increased intake of dietary acids, and a move away from formalised meal times, has been accompanied by a rise in the incidence of dental erosion and tooth wear. In view of this, oral care compositions which help prevent dental erosion and tooth wear would be advantageous.

WO 2004/054531 (Procter and Gamble) dicloses a method of enhancing fluoridation and mineralisation of teeth. These methods provide increased resistance to acid demineralisation, and involve the use of fluoride and phosphate containing polymeric material

WO94/26245(Church &Dwight) describes oral compositions comprising submicron or agglomerated submicron zinc oxide particles which particles have a primary particle size of less than 1 micron and an agglomerated secondary particle size of 50 microns or less, which compositions are said to exhibit enhanced antiplaque properties. It is suggested that zinc ions are released from the agglomerated zinc oxide particles trapped in the plaque when plaque bacteria metabolize sugars and release acids. These zinc ions are believed to inhibit nucleation of calcium phosphate crystals and thus prevent tartar from forming. Oral health compositions are described further comprising a source of fluoride ions.

EP-A-737470 (Quest) describes oral compositions comprising an anti-plaque agent such as a colloidal zinc oxide which may be coated with a polymer which contains carbohydrate or peptide structures which are specifically recognised by bacterial adhesins. Such agents can be targeted at specific sites in the human mouth where the antiplaque agent is then released at acidic pH. It is suggested that anti-caries agents such as fluoride salts may also be included.

WO 99/59539 (Boots) describes odour absorbing compositions including oral health compositions comprising high surface area zinc oxide and having a particle size diameter between 0.1 to 200 µm, preferably between 0.1 to 20.5 µm. Oral health compositions are described further comprising a source of fluoride ions.

WO 2007/013937 (Colgate) describes oral compositions comprising a zinc ion source such as zinc oxide in the form of non-aggregated nanoparticles which may have an average particle size between 1 and 250 nm. It is suggested that lower amounts of non-aggregated zinc nanoparticles can be used in comparison with a standard zinc ion source whilst maintaining or enhancing the beneficial effects of zinc which are stated to include antiplaque, anticalculus and deodorant properties. Oral health compositions are described further comprising a source of fluoride ions.

The role of zinc salts in protecting against dental caries is uncertain. Two in vitro studies have suggested that zinc can enhance the anticaries properties of oral compositions comprising a source of fluoride ions, whilst two other in vitro studies have reported contrary results.

A beneficial anticaries effect for zinc salts is described in US Patent 6120754 (Chesebrough Pond) which claims a method of remineralising teeth with a two phase oral care product providing a source of calcium, phosphate and fluoride ions which may optionally contain a zinc salt. Data is provided in Example 8B) suggesting that the inclusion of zinc citrate can enhance the degree of remineralisation (as measured by an increase in teeth hardness) of such a two phase oral care product in an in vitro caries model based upon a demineralisation and remineralisation cycling study.

Another positive study is reported in US Patent 6471946 (Sunstar) which describes a method for remineralising teeth having subsurface caries lesions using an oral composition comprising an isomalt disaccharide (palatinit) in combination with a remineralisation enhancing ingredient stated to be a fluoride, zinc, phosphorous or calcium compound or a mixture thereof. In table 4 data is provided suggesting that whilst zinc oxide in the absence of palatinit or a fluoride compound has minimal remineralising properties, increasing amounts of zinc oxide can enhance the remineralising ability of palatinit in combination with a fluoride compound in an in vitro caries model based upon a demineralisation and remineralisation cycling study. In table 5 are described dentifrices comprising sodium fluoride and small particle size zinc oxide having an average particle diameter ranging from 0.04 µm to 0.5 µm. It is stated that the particle diameter should be less than 0.3 µm, to lessen the astringency caused by zinc compounds.

In contrast with these positive in vitro results, Mellberg et al (J Dent Res 62(2): 145-147, 1983) previously reported that zinc citrate inhibits fluoride uptake by artificial caries lesions where it is postulated that the zinc salt forms insoluble zinc phosphate thereby coating hydroxyapatite surfaces and blocking fluoride uptake.

Furthermore White et al (Caries Res 1987: 21:40-47) have suggested a reduced fluoride uptake into demineralised enamel in the presence of zinc chloride or zinc citrate in an in vitro caries study.

Whilst these in vitro studies may suggest that zinc salts can have a positive or negative effect on the anti-caries efficacy of fluoride, two in vivo studies suggest that in the real world zinc may have little impact on fluoride activity.

This has been reviewed by Ingram et al (Adv Dent Res 8(2);158-165, 1994) who states that although zinc has been shown to absorb upon apatite material to restrict subsequent crystal growth it does not appear to affect the action of fluoride, including remineralisation, adversely. It is suggested this may be due to the fact that the uptake of zinc is reversible.

In this review Ingram et al note that despite the fact that treatment of hydroxyapatite with zinc salts can reduce mineral solubility in acid, there is no evidence that zinc plays a role in the caries process. In the rat, as reported by Ingram et al (J Dent Res 63: 491 1984), the anti-caries activity of fluoride was unaffected by the inclusion of zinc, and this was confirmed in humans by Stephen et al (Community Dent Oral Epidemiol 16: 321 -325, 1988).

Takatsuka et al (Dental Materials 2005, 21, 1170-1177) have reported that zinc oxide can inhibit dentine demineralisation and therefore may be effective in the prevention of root caries.

The role of zinc salts in protecting against dental erosion is described in WO 2004/054529 (Procter & Gamble) which claims a method of protecting against dental erosion comprising administering an oral care composition comprising a polymeric mineral surface active agent (such as a polyphosphate) and/or a source of metal ions selected from stannous, zinc and copper. It is stated that such metal ions form insoluble compounds or complexes that deposit on the tooth surface, thereby forming a film or coating that protects teeth from erosive damage immediately after use and for at least one hour thereafter. Many examples of suitable zinc salts are listed including zinc oxide, although zinc citrate and zinc lactate are stated to be particularly preferred. There is also a suggestion that such oral compositions may optionally comprise a source of fluoride ions.

In this P&G patent application the claimed use of such zinc salts in protecting against dental erosion is based upon an in vitro erosion cycling protocol which provides data in Table 3 suggesting that zinc citrate is slightly better than placebo in minimising the complete loss of mineral from the tooth surface following repeated acidic erosive challenges (10.7 µm for zinc citrate vs 13.9 µm for placebo - depth of complete mineral loss).

Beneficial effects of a zinc salt against an acidic erosive challenge in vitro have also been reported by Brudevold et al (Arch. oral Biol., 8: 135-144, 1963) which presents data in Table 5 on page 141 demonstrating that zinc acetate is almost as effective as sodium fluoride in decreasing acid dissolution of hydroxyapatite when challenged with an acidic acetate buffer at pH4.5.

The present invention is based on the discovery that nano particulate zinc oxide can combat dental erosion and can also be combined with a source of fluoride ions without adversely impacting upon the delivery of fluoride to dental enamel.

This is surprising given the mechanism of action suggested in WO2004/054529 for the acid protection effects of zinc salts, said to involve the formation of a substantive coating or film on the tooth surface, which according to Mellberg et al might be expected to interfere with the uptake of fluoride into enamel.

It appears that the nano particulate zinc oxide is able to provide enamel with a protective barrier against dietary acid which can add to the anti-erosion efficacy of fluoride (known to enhance remineralisation and decrease demineralisation of dental enamel). Teeth are strengthened by the dual actions of the nano particulate zinc oxide and source of fluoride ions.

Furthermore, whilst WO 2004/054529 suggests that any water soluble or sparingly soluble zinc salt can be used to protect teeth against erosion, the data presented in table 3 thereof demonstrates that zinc citrate, stated to be a particularly preferred zinc salt, is actually rather poor and, as noted above, only slightly better than placebo in providing any effective protection. Therefore the skilled addressee of this prior published document would expect less preferred zinc salts, including zinc oxide, to be even less effective in protecting teeth against an acidic erosive challenge.

In addition it has now been discovered that particulate zinc oxide in the absence of palatinit can combat dental caries and can advantageously be combined with a source of fluoride ions. It appears that the particulate zinc oxide is able to provide enamel with a protective barrier against bacterial acid which can add to the known anti-caries efficacy of fluoride. Teeth are strengthened by the dual actions of the particulate zinc oxide and a source of fluoride ions.

### SUMMARY OF THE INVENTION

Accordingly, the present invention provides an oral care composition according to claim 1.

### DETAILED DESCRIPTION OF THE INVENTION

Zinc oxide is in nanoparticulate form having a mean particle diameter less then 1µm, for example in the ranges from 1 to 750nm, from 2nm to 500nm, from 5nm to 250nm and from 10 to 100nm.

The zinc oxide may be present in an amount of 0.001 to 10.0% by weight of the total composition, suitably from 0.01 to 5%, for example from 0.1 to 3.0% by weight of the total composition.

Zinc oxide is formulated together with a dispersing agent which can adsorb onto the surface of the zinc oxide particles to provide steric or ionic barriers so to help prevent their agglomeration or aggregation. Suitable dispersing agents are surfactants including solubilising or wetting agents or water-soluble polymers such as polyelectrolytes.

Suitably the dispersing agent is selected from a polyol (for example glycerine, propylene glycol, polyethylene glycol, polyvinyl alcohol, sorbitol mannitol, or xylitol), a polyvinylpyrrolidone (PVP) or derivative thereof (for example a vinylpyrrolidone vinyl acetate copolymer or a vinylpyrrolidone vinyl alcohol copolymer), a polyphosphate (for example a cyclic phosphate such as sodium hexametaphosphate) or a water soluble copolymer having polymer segments attractive to zinc oxide particles and having polymer segments that render them water-soluble (for example water soluble copolymers of the type described in WO2004/000916 (Nanophase)). Examples of suitable dispersing agents include propylene glycol, PVP or sodium hexametaphosphate.

Commercially available nanoparticulate zinc oxide aqueous suspensions are available from Nanophase Technologies, 1319 Marquette Drive, Romeoville, IL 60446. USA, examples of which are Nanoshield ZN-3008C and Nanoshield ZN-3014A which are nanoparticulate zinc oxide suspensions respectively stabilised with a cationic or anionic dispersing agent.

Suitably the oral care composition further comprises a source of fluoride ions.

The combination of the zinc oxide and a source of fluoride ions is not only able to strengthen and thereby protect teeth from an acidic erosive or caries challenge but also is able to reharden enamel softened by an acidic erosive or caries challenge.

Examples of a source of fluoride ions for use in the present invention include an alkali metal fluoride such as sodium fluoride, an alkali metal monofluorophosphate such a sodium monofluorophosphate, stannous fluoride, or an amine fluoride in an amount to provide from 25 to 3500pm of fluoride ions, preferably from 100 to 1500ppm. A suitable fluoride source is an alkali metal fluoride such as sodium fluoride, for example the composition may contain 0.1 to 0.5% by weight of sodium fluoride, eg 0.205% by weight (equating to 927ppm of fluoride ions), 0.2542% by weight (equating to 1150ppm of fluoride ions) or 0.315% by weight (equating to 1426ppm of fluoride ions).

Compositions of use in the present invention do not contain an isomalt disaccharide, referred to as palatinit in the above-noted Sunstar US patent.

Compositions of use in the present invention may further comprise a desensitising agent for combating dentine hypersensitivity. Examples of desensitising agents include a tubule blocking agent or a nerve desensitising agent and mixtures thereof, for example as described in WO 02/15809. Suitable desensitising agents include a strontium salt such as strontium chloride, strontium acetate or strontium nitrate or a potassium salt such as potassium citrate, potassium chloride, potassium bicarbonate, potassium gluconate and especially potassium nitrate.

A desensitising amount of a potassium salt is generally between 2 to 8% by weight of the total composition, for example 5% by weight of potassium nitrate can be used.

Compositions of use in the present invention will contain appropriate formulating agents such as abrasives, surfactants, thickening agents, humectants, flavouring agents, sweetening agents, opacifying or colouring agents, preservatives and water, selected from those conventionally used in the oral care composition art for such purposes. Examples of such agents are as described in EP 929287.

Compositions of use in the present invention are typically formulated in the form of toothpastes, sprays, mouthwashes, gels, lozenges, chewing gums, tablets, pastilles, instant powders, oral strips and buccal patches.

Compositions of use in the present invention may be prepared by admixing the ingredients in the appropriate relative amounts in any order that is convenient and if necessary adjusting the pH to give a desired value for example from 5.5 to 9.0

In a further aspect the zinc oxide may be incorporated into a dentifrice composition of the type described in WO2006/100071.

Accordingly the present invention further provides a first dentifrice composition which composition comprises particulate zinc oxide as hereinbefore described, a fluoride ion source as hereinbefore described and a silica dental abrasive, the dentifrice having a Relative Dentine Abrasivity (RDA) value from 20 to 60 and a pH in the range 5.5 to 9.0 and being free of an orthophosphate buffer or a water-soluble salt of a C₁₀₋₁₈ alkyl sulphate.

The pH referred to is that measured when the dentifrice composition is slurried with water in a 1:3 weight ratio of the composition to water.

The nano particulate zinc oxide is formulated together with a dispersing agent as hereinbefore described.

Suitably the first dentifrice composition of the present invention does not include a calcium salt which can reduce the availability of free fluoride ions.

Examples of suitable silica dental abrasives include those marketed under the following trade names Zeodent, Sident, Sorbosil or Tixosil by Huber, Degussa, Ineos and Rhodia respectively. The silica abrasive should be present in an amount sufficient to ensure the RDA of the dentifrice is between 20 and 60, for example between 25 and 50 or between 25 and 40 to ensure adequate cleaning of teeth by the dentifrice whilst not promoting abrasion of teeth, especially teeth suffering from dental erosion or having been softened by an acidic challenge.

The silica abrasive is generally present in an amount up to 15% by weight of the total composition, for example from 2 to 10% by weight, generally at least 5% for example from 5 to 7% by weight, suitably 6% by weight of the total composition. Reducing the level of silica abrasive has the advantage of not only lowering the abrasivity of the dentifrice but also minimising any interaction of the abrasive (or trace amounts of contaminants in the abrasive) with fluoride ions thereby increasing the availability of free fluoride ions.

Suitable surfactants for use in the first dentifrice composition of the present invention include amphoteric surfactants for example, long chain alkyl betaines, such as the product marketed under the tradename 'Empigen BB' by Albright & Wilson, and preferably long chain alkyl amidoalkyl betaines, such as cocamidopropylbetaine, or low ionic surfactants such as sodium methyl cocoyl taurate, which is marketed under the trade name Adinol CT by Croda, or mixtures thereof. An amphoteric surfactant can be used alone as sole surfactant or can be combined with a low ionic surfactant.

Suitably, the surfactant is present in the range 0.1 to 10%, for example 0.1 to 5% such as from 0.5 to 1.5% by weight of the total composition.

Suitable thickening agents include, for instance, nonionic thickening agents such as, for example, (C1-6)alkylcellulose ethers, for instance methylcellulose; hydroxy(C 1-6)alkylcellulose ethers, for instance hydroxyethyl cellulose and hydroxypropylcellulose; (C2-6)alkylene oxide modified (C1-6)alkylcellulose ethers, for instance hydroxypropyl methylcellulose; and mixtures thereof. Other thickening agents such as natural and synthetic gums or gum like material such as Irish Moss, xanthan gum, gum tragacanth, carrageenan, sodium carboxymethylcellulose, polyvinylpyrrolidone, polyacrylic acid polymer (carbomer), starch and thickening silicas may also be used. Suitably the thickening agent is mixture of a thickening silica and xanthan gum, optionally with carrageenan and/or a carbomer.

Suitably the thickening agent is present in the range 0.1 to 30%, for example from 1 to 20%, such as from 5 to 15% by weight of the total composition.

Suitable humectants include for instance, glycerin, xylitol, sorbitol, propylene glycol or polyethylene glycol, or mixtures thereof; which humectant may be present in the range from 10 to 80%, for example from 20 to 60%, such as from 25 to 50% by weight of the total composition.

In order to combat dentine hypersensitivity the first dentifrice composition of the present invention may further comprise a desensitising agent as hereinbefore described, especially potassium nitrate. The presence of potassium nitrate advantageously may provide an enhanced stain removal effect, which is of particular benefit for low abrasivity formulations, which otherwise might be expected to have relatively low cleaning performance.

The pH of the first dentifrice composition of the present invention may be in the range 6.0 to 8.0, suitably from 6.5 to 7.5 and can be adjusted by the incorporation of a base such as sodium hydroxide.

In a further aspect the present invention provides a second dentifrice composition comprising particulate zinc oxide as hereinbefore described, a fluoride ion source as hereinbefore described (for example an alkali metal fluoride), a thickening system comprising a thickening silica in combination with xanthan gum optionally with carrageenan and/or a carbomer, an anionic surfactant (for example a water-soluble salt of a C₁₀₋₁₈ alkyl sulphate such as sodium lauryl sulphate) and a silica dental abrasive in an amount up to 20% (suitably from 5 to 20% for example from 10 to 16%) by weight of the total composition, the dentifrice having a pH in the range from 5.5 to 9.0 (suitably from 6.0 to 8.5, for example from 6.5 to 7.5), and being free of an orthophosphate buffer or a calcium salt. If desired such a dentifrice composition may also comprise a desensitising agent as hereinbefore described.

The invention is further illustrated by the following Examples.

| **Dentifrice Formulation** | **Ex 1** | **Ex 2** | **Ex A** | **Ex B** |
|---|---|---|---|---|
| Raw Material | %w/w | %w/w | %w/w | %w/w |
| Sorbitol 70% solution - humectant | 30.000 | 30.000 | 30.000 | 30.000 |
| Abrasive silica | 6.000 | 6.000 | 6.000 | 6.000 |
| Thickening silica | 12.000 | 12.000 | 12.000 | 12.000 |
| Glycerin - humectant | 8.000 | 8.000 | 8.000 | 8.000 |
| Polyethylene glycol - humectant | 3.000 | 3.000 | 3.000 | 3.000 |
| Cocoamidopropylbetaine - surfactant | 1.200 | 1.200 | 1.200 | 1.200 |
| Xanthan gum - thickener | 0.800 | 0.800 | 0.800 | 0.800 |
| Titanium dioxide - opacifier | 0.100 | 0.100 | 0.100 | 0.100 |
| Potassium Nitrate - active | 5.000 | 5.000 | 5.000 | 5.000 |
| Sodium Fluoride - active | 0.000 | 0.243 | 0.243 | 0.000 |
| Nanoparticulate Zinc Oxide- active | 0.500 | 0.500 | 0.000 | 0.000 |
| Flavour oil | 1.100 | 1.100 | 1.100 | 1.100 |
| Saccharin sodium - sweetener | 0.300 | 0.300 | 0.300 | 0.300 |
| Sodium Hydroxide - pH adjuster | 0.082 | 0.272 | 0.082 | 0.082 |
| Water | To 100 | To 100 | To 100 | To 100 |
| pH | 7.1 | 7.1 | 7.1 | 7.1 |

Examples 1 and 2 are dentifrices of the present invention containing nanoparticulate zinc oxide (having a mean particle diameter in the range of 50 to 70 nm), the latter example also containing a source of fluoride ions. Dentifrices A and B are comparative examples excluding nanoparticulate zinc oxide, the latter example also excluding a source of fluoride ions.

### Example 3

### Enamel Surface Softening Microhardness Study for assessing the efficacy of nanoparticulate zinc oxide as an anti-erosion agent, in the presence and absence of fluoride

The study performed involved incubation of human enamel in 1 % citric acid (pH 3.8), following a 2 minute treatment with one of the four dentifrice formulations listed above, Example 1 (nano ZnO), Example 2 (nano ZnO + Fluoride), Comparative Example A (Positive Control containing Fluoride) and Comparative Example B (Negative Control excluding nano ZnO and Fluoride). Specimens were washed with water in between treatment and erosion procedures. Acid exposure times investigated were 10, 20 and 30 minutes, and microhardness measurements were made at each time point, after washing the specimens thoroughly with distilled water. Six indents were obtained for each enamel specimen at all time points, including at baseline. Each treatment was performed on six individual specimens (n=6).

The results of the softening study are summarised in Graph 1 and Table 1. The values for enamel hardness have been normalised relative to the individual baseline microhardness values, thus data at subsequent time points reflects softening of the enamel. The error bars in Graph 1 represent standard deviations.

The nano zinc oxide and/or fluoride containing dentifrice treatments (Examples 1, 2 and A) were statistically superior to the placebo dentifrice (Example B) containing neither of the active ingredients, in the prevention of citric acid induced enamel surface softening at the 20 and 30 minute time points in the experiment. After 20 minutes acid exposure, the protection offered by dentifrices of Examples 1, 2 and A was statistically equivalent. After 30 mins acid exposure, treatment with the dentifrice of Example A was directionally but not statistically superior to treatment with either of the dentifrices of Examples 1 and 2, which were statistically equivalent to one another in their protection at this time point in this *in vitro* model. After 10 minutes acid treatment, there were statistically significant differences between the enamel treated with fluoride containing dentifrices of Examples 2 and A compared to enamel treated with the placebo dentifrice, Example B. Examples 2 and A were statistically equivalent to one another and directionally superior to the dentifrice of Example 1.

Overall these data suggest that a dentifrice containing nanoparticulate zinc oxide without fluoride offers similar protection against an acidic erosive challenge to a fluoride containing dentifrice. Furthermore the efficacy of a fluoride containing dentifrice against dental erosion does not appear to be significantly compromised by the inclusion of nano zinc oxide which is therefore able to provide enamel with a protective barrier against dietary acid without adversely impacting upon the uptake of fluoride into dental enamel.

### Graph 1. Relative microhardness values for human enamel exposed to citric acid following treatment with dentifrice formulations of Examples 1, 2, A or B.

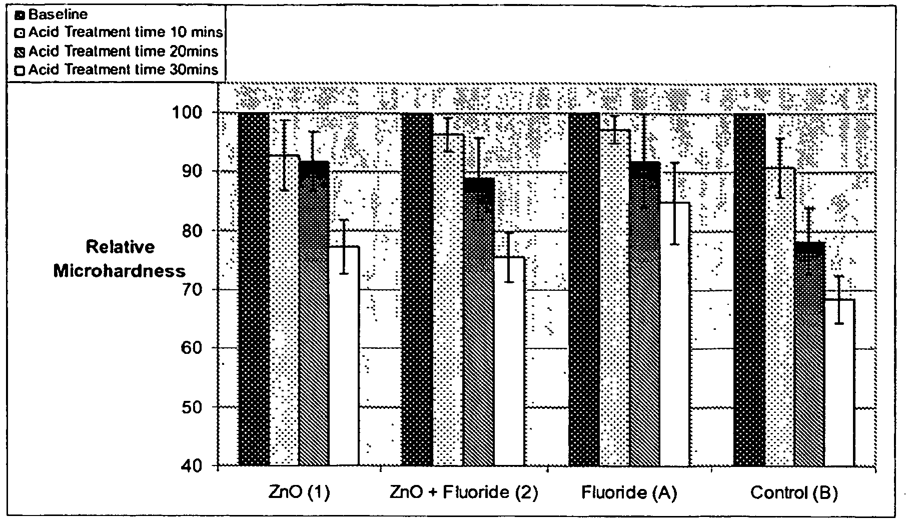

**Table 1. Relative microhardness values for human enamel exposed to citric acid following treatment with dentifrice formulations of Examples 1, 2, A or B.**

| | **% VHN compared to baseline** | | | |
|---|---|---|---|---|
| **Test Agent** | **Baseline** | **10 minutes** | **20 minutes** | **30 minutes** |
| 0.50% ZnO (Ex 1) | 100 | 93 | 92 | 77 |
| 0.50% ZnO +300 ppm F (Ex 2) | 100 | 96 | 89 | 75 |
| 300 ppm F (Ex A) | 100 | 97 | 92 | 85 |
| Control (Ex B) | 100 | 91 | 78 | 68 |

### Example 4

### Hydroxyapatite Microplate Method for comparing the effect of stabilised suspensions of nanoparticulate zinc oxide, Nanoshield ZN-3008C and Nanoshield ZN-3014A, as anti-erosion agents, in the presence and absence of fluoride

The wells of 96-well 200µl microplates were filled with a concentrated suspension of high resolution hydroxyapatite (HA) powder (20g HA in 200ml acetone). The plates were allowed to dry under agitation (50 rpm on an orbital shaker) and then washed thoroughly with deionised water to remove any loose HA. For erosion experiments, the wells of the HA coated microplates were filled with deionised water and left to hydrate for 60 mins. The water was then removed and each of the 8 wells of one lane of the microplate filled with 200µl of one of the agents to be tested. Each plate contained 12 lanes, so 6 agents could be tested in duplicate (at n=8) in each microplate. The actives were left in the wells for 30 mins under agitation, after which time the plates were again washed thoroughly with deionised water. The erosive challenge used for the study was an orange juice mimic, 1% citric acid, pH 3.8. The wells of the plate were exposed to 200µl of the citric acid solution under agitation for 30 mins. After the acid challenge, 50 µl of the citric acid was removed from each well and placed into the corresponding well of a new 96-well microplate. 50 µl of vanadomolybdate reagent was then added to each well and after 5 mins, the absorbance of the solution in each well at 450nm was read using a microplate plate reader. The test agents assessed using this technique were nanoparticulate zinc oxide aqueous suspension, 0.5 wt% in water, stabilised with both anionic and cationic dispersants (Nanoshield ZN-3008C and Nanoshield ZN-3014A respectively, both 70nm average particle size and obtained from Nanophase Technologies, 1319 Marquette Drive, Romeoville, IL 60446. USA).

Graph 2 displays the data for the microplate erosion study. The chart contains data from one microplate, where treatments have been carried out in duplicate at n=8 each time, as described above. The effects of cationic and anionic stabilised nanoparticulate ZnO have been examined, in the presence and absence of fluoride. Where fluoride is present, the 0.5% suspensions have been prepared in 300ppm fluoride solutions (using sodium fluoride). The positive control used was 300ppm fluoride, and the negative control was deionised water.

The results clearly show that the nanoparticulate zinc oxide, stabilised by the cationic or ionic dispersants, are approximately equivalent to 300ppm fluoride at preventing the demineralisation of hydroxyapatite in this in vitro model. In addition, when the nanoparticulate zinc oxide is present along with fluoride in solution, the effect of the two anti-erosion actives is additive, indicating that the zinc oxide is not adversely interfering with the uptake of fluoride into the hydroxyapatite.

### Graph 2. Hydroxyapatite microplate erosion data to assess the anti-erosion efficacy of stabilised suspensions of nanoparticulate zinc oxide, in the presence and absence of fluoride.

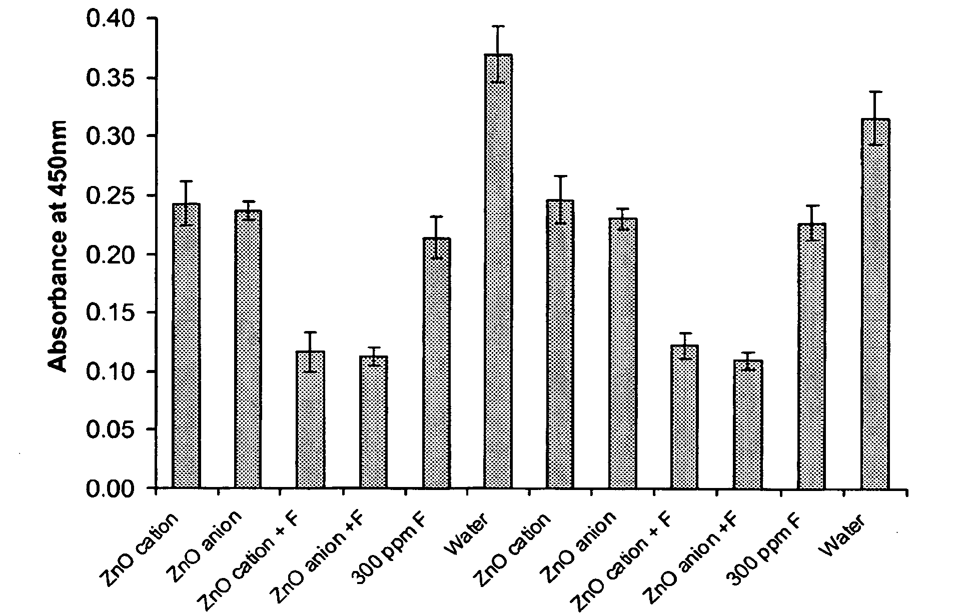

### Example 5

### Hydroxyapatite Microplate Method for assessing the efficacy of HMP stabilised suspensions of nanoparticulate zinc oxide as an anti-erosion agent, in the presence and absence of fluoride

An additional experiment was performed as described in Example 4, where the stabilising agent for the nanoparticulate zinc oxide (0.5 wt% suspension in water) was sodium hexametaphosphate (HMP) present in an amount of 2 wt% with respect to the zinc oxide (le present in an amount of 0.01 wt%). Graph 3 displays the data obtained. The nanoparticulate zinc oxide was tested in the presence and absence of fluoride again, and at two different pH values (pH 7 and pH 9). The data show that while the nanoparticulate zinc oxide alone at pH 7 or 9 is statistically superior to water at preventing demineralisation of hydroxyapatite (and statistically inferior to 225ppm fluoride alone), the combinations of the zinc oxide and 225ppm fluoride are statistically superior to 225 ppm fluoride alone in this regard. At pH 7, the combination is directionally superior to the combination at pH 9.

### Graph 3. Hydroxyapatite microplate erosion data to assess the anti-erosion efficacy of HMP stabilised suspensions of nanoparticulate zinc oxide, in the presence and absence of fluoride.

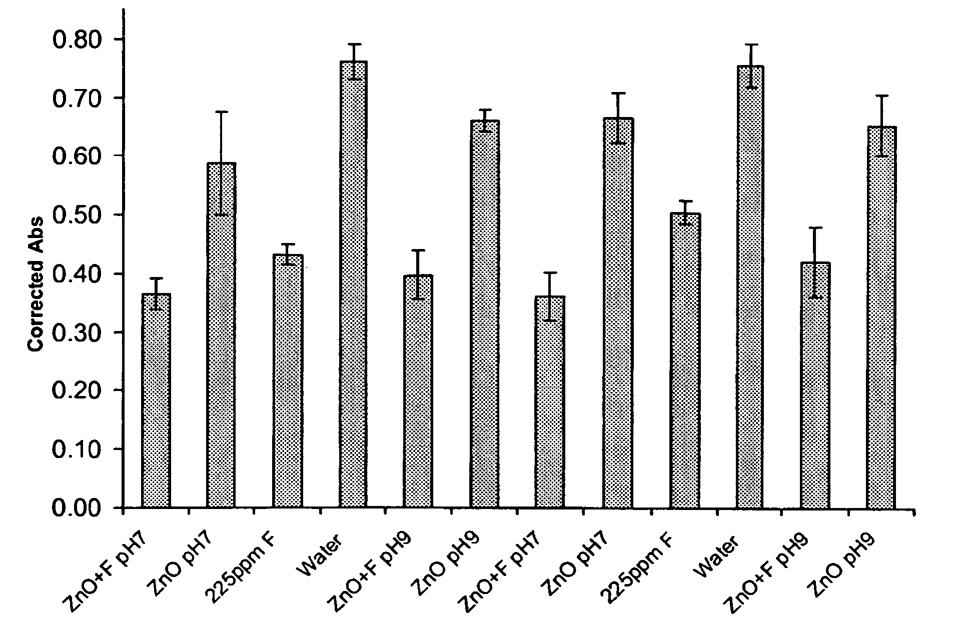

### Example 6

### 20-Day In vitro Erosion Cycling Study

Human enamel specimens (3 x 3 mm) were ground and polished using silicon carbide paper. The top side of the specimens was ground using 1200-grit paper and then serially polished using 4000-grit paper followed by 1-mm diamond polishing suspension. Each specimen was mounted on the end of an acrylic rod (1/4" diameter x 2" long) using superglue. The sides of each specimen were covered with an epoxy resin so that only the enamel surface was exposed. Eighteen specimens per group were used for the study. Each group was composed of 3 subgroups of 6 specimens. Initial hardness of the sound specimens was determined using a Vickers hardness indenter by applying a load of 200g for 15 seconds. The average specimen microhardness was determined from four indentations on the surface of each specimen. Only specimens with a sound surface hardness of between 300 and 350 were accepted. A 50:50 mixture of pooled, human saliva and a mineral solution was used as the remineralisation medium in all treatment regimens, and the demineralising solution was 1% citric acid, pH 3.8. The four treatments considered were: Group A) Deionised water, Group B) 300ppm Fluoride, Group C) 0.50wt % ZnO in deionized water, Group D) 0.50wt % ZnO in 300ppm Fluoride. The fluoride source used was sodium fluoride and the zinc oxide was Nanoshield ZN-3008C as described above in Example 4. The cyclic treatment regimen consisted of five, two-minute acid challenges in the demineralizing solution and three, two-minute treatment periods (with stirring at 350 rpm). After each treatment, the specimens were rinsed with running deionized water and then placed back into the saliva remineralization system. This regimen was repeated for 10 days, after which time the specimens were analysed by remeasuring their microhardness. A further 10 days of cycling was then performed and a 20 day microhardness value measured. The treatment schedule for this experiment is given in Table 2 and the data obtained is displayed in Graph 4 and Table 3 below.

**Table 2**

| | |
|---|---|
| 8:00-8:02 a.m. | Treatment 1* |
| 8:02-10:00 a.m. | Saliva treatment |
| 10:00-10:02 a.m. | Acid challenge 1 (1.0% citric acid, pH 3.75) |
| 10:02-11:00 p.m. | Saliva treatment |
| 11:00-11:02 p.m. | Acid challenge 2 (1.0% citric acid, pH 3.75) |
| 11:02-12:00 p.m. | Saliva treatment |
| 12:00-12:02 p.m. | Treatment 2 |
| 12:02-1:00 p.m. | Saliva treatment |
| 1:00-1:02 p.m. | Acid challenge 3 (1.0% citric acid, pH 3.75) |
| 1:02-2:00 p.m. | Saliva treatment |
| 2:00-2:02 p.m. | Acid challenge 4 (1.0% citric acid, pH 3.75) |
| 2:02-3:00 p.m. | Saliva treatment |
| 3:00-3:02 p.m. | Acid challenge 5 (1.0% citric acid, pH 3.75) |
| 3:02-4.30 p.m. | Saliva treatment |
| 4:30-4.32 p.m. | Treatment 3 |
| 4:32p.m.-8:00 a.m. | Saliva treatment |

### Graph 4. 20-day in vitro cycling erosion data to assess the anti-erosion efficacy of stabilised suspensions of nanoparticulate zinc oxide, in the presence and absence of fluoride.

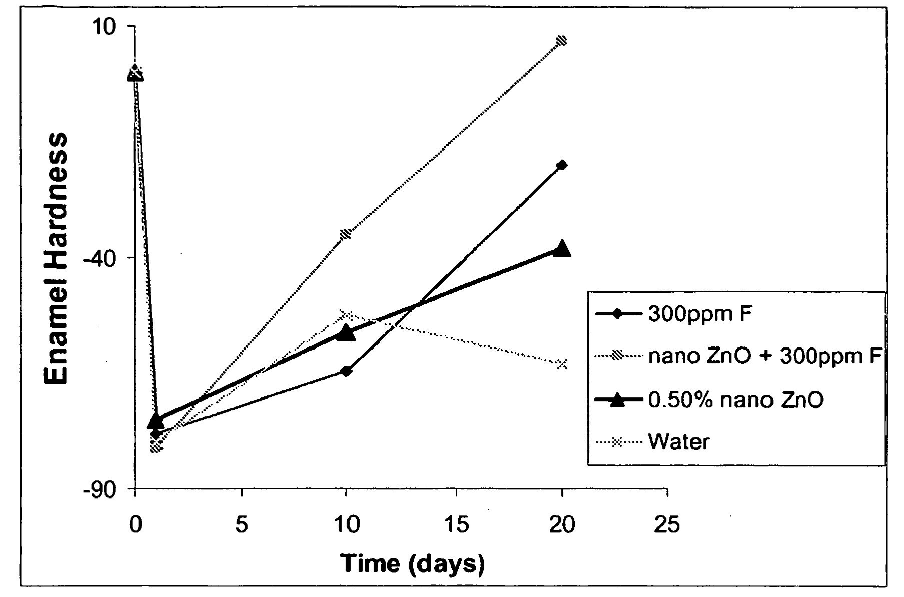

Statistical analysis of the data above in Graph 4 was performed with a one-way analysis of variance using Sigma Stat (3.0) Software. ANOVA indicated a significant difference so pair wise comparisons between the groups was conducted (Student Newman Keuls test). The statistical analysis showed that the combination of the nanoparticulate zinc oxide and fluoride was superior to fluoride alone (or to zinc oxide alone) in preventing the demineralisation/promoting the remineralisation of artificial erosive lesions in human enamel.

**Table 3. 20-day in vitro cycling erosion data to assess the anti-erosion efficacy of stabilised suspensions of nanoparticulate zinc oxide, in the presence and absence of fluoride.**

| **Test Agent** | | **Sound Enamel** | **Lesion Baseline** | **Post 10 days** | **Change at 10 days** | **Post 20 days** | **Change at 20 days** |
|---|---|---|---|---|---|---|---|
| 0.50% ZnO+ 300 ppm F | Mean | 333.7 | 252.6 | 217.3 | -35.3 | 259.1 | 6.5 |
| | SD | 9.5 | 23.6 | 48.1 | 40.7 | 22.0 | 24.5 |
| | SEM | 2.3 | 5.7 | 11.7 | 9.9 | 5.3 | 5.9 |
| 300 ppm F | Mean | 332.3 | 254.1 | 189.5 | -64.6 | 234.0 | -20.1 |
| | SD | 9.7 | 28.3 | 73.6 | 75.9 | 26.8 | 20.5 |
| | SEM | 2.3 | 6.7 | 17.3 | 17.9 | 6.3 | 4.8 |
| 0.50% ZnO | Mean | 330.9 | 255.9 | 199.7 | -56.2 | 215.5 | -37.9 |
| | SD | 15.0 | 26.2 | 31.0 | 30.3 | 17.1 | 23.1 |
| | SEM | 3.6 | 6.4 | 7.5 | 7.3 | 4.3 | 5.8 |
| DI water | Mean | 333.4 | 253.4 | 201.0 | -52.4 | 190.3 | -63.1 |
| | SD | 10.0 | 31.1 | 30.7 | 25.8 | 24.8 | 30.0 |
| | SEM | 2.4 | 7.5 | 7.4 | 6.3 | 6.0 | 7.3 |

### Example 7

### Hydroxyapatite Microplate Method for comparing the effect of various zinc compounds as anti-erosion agents, in the presence and absence of fluoride.

The experiments were performed as described in Example 4 above, using one side of a hydroxyapatite microplate (n=8). The test agents assessed in this case were a) 0.5 wt% Nanoshield ZN-3008C in water, b) 0.5 wt% zinc acetate, c) 0.5 wt% zinc citrate and d) 0.5 wt% zinc oxide (bulk material). For the first experiment these zinc compounds were prepared in deionised water and for the second experiment they were prepared in 225ppm fluoride solution (using sodium fluoride). Positive and negative controls used were 225ppm fluoride and deionised water respectively.

### Graph 5. Hydroxyapatite microplate erosion data to assess the anti-erosion efficacy of various zinc compounds.

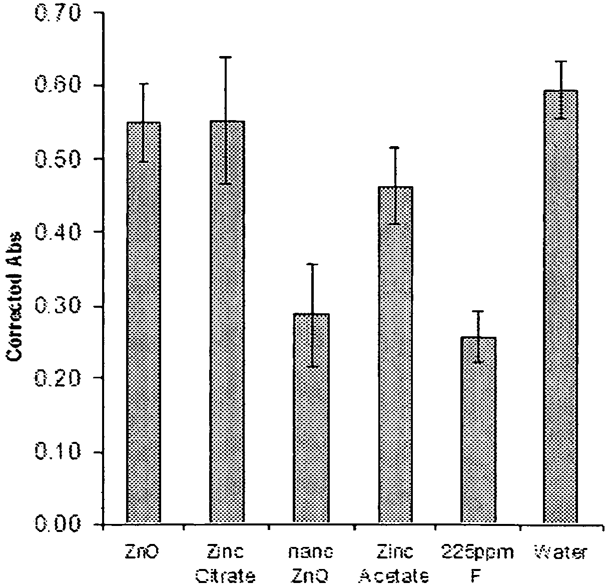

Graphs 5 and 6 contain data from experiments carried out on the zinc compounds alone (Graph 5), and in combination with 225ppm fluoride (Graph 6). The data clearly show that the nanoparticulate zinc oxide, stabilised by the cationic dispersant, is statistically equivalent to 225ppm fluoride at preventing the demineralisation of hydroxyapatite in this in vitro model. In addition, when the nanoparticulate zinc oxide is present along with 225ppm fluoride in solution, the effect of the two agents is additive as shown in Graph 6. From Graph 5 above, it would appear that the only other zinc compound tested (in the absence of fluoride) to provide any benefit vs water was zinc acetate, which was statistically inferior to both the stabilised nanoparticulate zinc oxide and 225ppm fluoride, but statistically superior to water in preventing demineralisation of hydroxyapatite. Graph 6 shows that the combination of zinc acetate and 225ppm fluoride is directionally superior but statistically equivalent to 225ppm fluoride alone. Zinc citrate and zinc oxide (bulk material) in combination with fluoride are statistically superior to water but statistically inferior to fluoride alone in preventing the demineralisation of hydroxyapatite, suggesting a detrimental effect of these two zinc salts on the activity of fluoride.

### Graph 6. Hydroxyapatite microplate erosion data to assess the anti-erosion efficacy of various zinc compounds in the presence of fluoride.

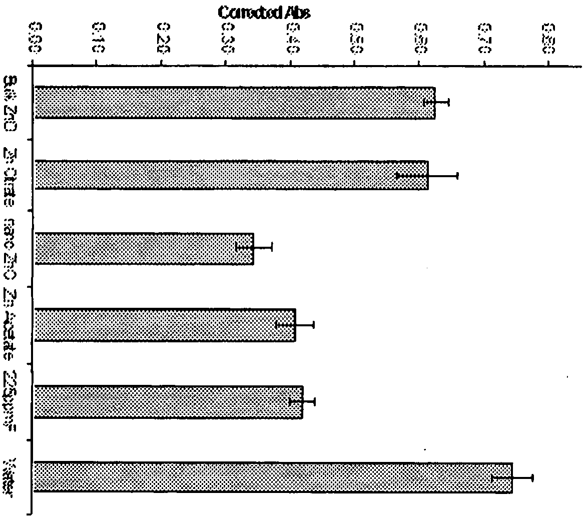

### Example 8

### Hydroxyapatite Microplate Method for assessing the efficacy of PVP and Propylene glycol stabilised suspensions of nanoparticulate zinc oxide as anti-erosion agents, in the presence of fluoride.

A final hydroxyapatite microplate experiment was performed as described in Examples 4 and 5 where the stabilising agent for the nanoparticulate zinc oxide was either polyvinyl pyrrolidone (PVP) or Propylene glycol. Graph 7 displays the data obtained. The nanoparticulate zinc oxide was tested in the presence of fluoride in each case. The data shows that nanoparticulate zinc oxide stabilised with PVP, in combination with fluoride, is statistically superior to either fluoride alone or water in preventing the demineralisation of hydroxyapatite. However, when the nanoparticulate zinc oxide is stabilised with propylene glycol, the combination of the zinc oxide and fluoride is statistically superior to water but only directionally superior to fluoride alone in this regard. Polyvinyl pyrrolidone in combination with fluoride (no nanoparticulate zinc oxide) is statistically inferior to fluoride alone in preventing demineralisation, and statistically equivalent to water. The ZN-3008C material in combination with fluoride was included in this study for comparison, and outperformed all other test agents/combinations.

### Graph 7. Hydroxyapatite microplate erosion data to assess the anti-erosion efficacy of PVP and Propylene glycol stabilised suspensions of nanoparticulate zinc oxide, in the presence of fluoride.

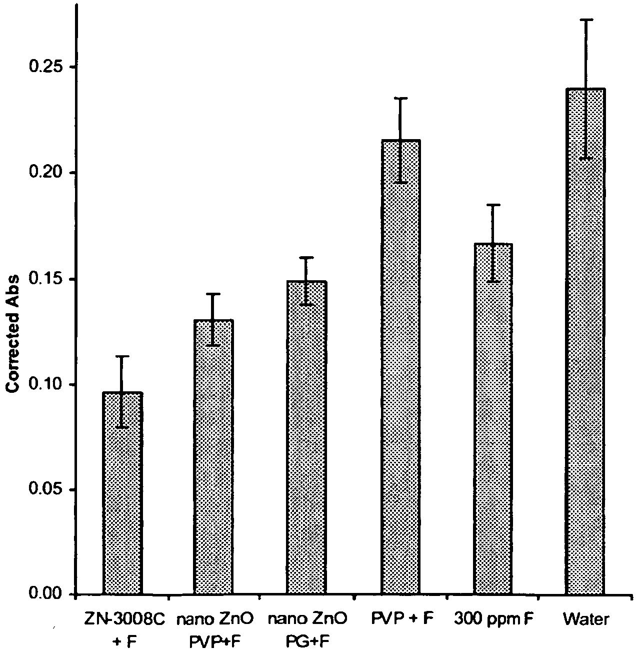

### Example 9 (Comparative Example)

### Hydroxyapatite Solubility Reduction Method (HASR) for comparing the effect of stabilised suspensions of nanoparticulate zinc oxide as anti-caries agents, in the presence and absence of fluoride.

50mg quantities of 'High Resolution' Hydroxyapatite (HA) were treated for 2 minutes with 5ml of a suspension of test agent adjusted to pH 7. The suspension was filtered and washed twice with 5ml of deionised water. The HA was further challenged with 0.1 M lactic acid, pH 4.5 for 2 minutes. The samples were filtered and the filtrate collected and diluted 1:10 with deionised water. The quantity of o-phosphate produced was determined by ion chromatography.

Graph 8 and Table 4 contains the data from the HASR anti-caries study. The effects of the cationic and anionic stabilized nanoparticulate ZnO (as described in Example 4) have been examined, in the presence and absence of 300 ppm fluoride. The positive control used was 300 ppm fluoride and the negative control was deionised water.

### Graph 8. Hydroxyapatite solubility reduction data to assess the anti-caries efficacy of stabilised suspensions of nanoparticulate zinc oxide, in the presence and absence of fluoride.

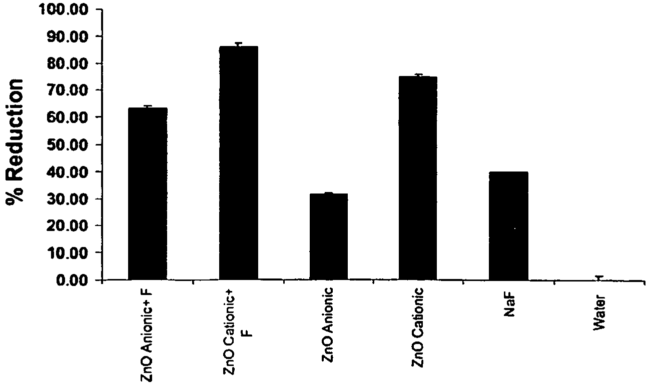

**Table 4. Hydroxyapatite solubility reduction data to assess the anti-caries efficacy of stabilised suspensions of nanoparticulate zinc oxide, in the presence and absence of fluoride.**

| **Treatment** | **% Reduction in HA Solubility** | **SEM** |
|---|---|---|
| Water | 0.00 | 1.61 |
| 300ppm Fluoride | 39.78 | 0.24 |
| ZnO (Anionic) | 31.50 | 0.65 |
| ZnO (Cationic) | 74.92 | 0.83 |
| ZnO + 300ppm Fluoride (Anionic) | 66.36 | 0.66 |
| ZnO + 300ppm Fluoride (Cationic) | 85.93 | 1.55 |

The results clearly show that the nanoparticulate zinc oxide stabilised by either cationic or anionic dispersants, are statistically superior to the negative control, in addition cationic stabilised nanoparticulate zinc oxide is also statistically superior to fluoride at preventing the demineralisation of hydroxyapatite in this in vitro model. When the nano particulate zinc oxide is present along with fluoride in solution, the effect of the two anti-caries actives is additive, indicating that zinc oxide is not adversely interfering with the uptake of fluoride into the hydroxyapatite.

### Example 10 (Comparative Example)

### Enamel Anti Caries Softening Microhardness Study for assessing the efficacy of nanoparticulate zinc oxide as an anti-cariogenic agent, in the presence and absence of fluoride

The study performed involved incubation of human enamel in 0.1 M lactic acid buffer (pH 4.5), following a 2 minute treatment with one of the following four formulations: (A) 0.5% suspension of nano ZnO. (B) 0.5% suspension of nano ZnO made up with 300ppm fluoride (sodium fluoride). (C) 300ppm fluoride (sodium fluoride) solution. (D) de-ionised water. The zinc oxide used was Nanoshield ZN-3008C as described above in Example 4. Specimens were washed with water in between treatments and erosion procedures. The acid exposure time was two hours, after which the specimens were rinsed in de-ionised water and re-treated with the appropriate formulation for 2 minutes. The specimens then were placed into the lactic acid for a further two hours. After the acid challenge they were washed thoroughly with de-ionised water and the micro-hardness measured. Six indents were obtained for each enamel specimen and each treatment was performed on six individual specimens (n=6).

The results of the softening study are summarised in Graph 9 and Table 5. The values for enamel hardness have been normalised relative to the individual baseline microhardness values, thus data at subsequent time points reflects softening of the enamel. The error bars in Graph 9 represent standard deviations.

### Graph 9. Relative microhardness values for human enamel exposed to a cariogenic lactic acid buffer, following treatment with nano zinc oxide suspension, nano zinc oxide suspension with fluoride solution, fluoride solution and water.

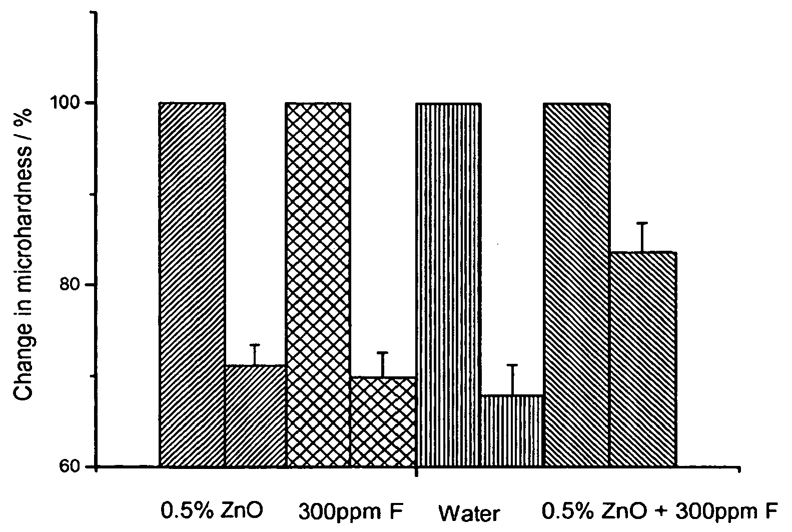

The results show that 0.5% nano zinc oxide suspension gives similar protection to that of 300ppm fluoride solution, both providing approximately 70% protection from the lactic acid buffer challenge. However, 0.5% nano zinc oxide suspension combined with 300ppm fluoride solution gives a significant protection benefit offering about 84% protection from the lactic acid buffer challenge.

Overall these data suggest that a formulation containing nanoparticulate zinc oxide without fluoride offers similar protection against a cariogenic challenge to a fluoride containing solution. Furthermore the efficacy of a nanoparticulate zinc oxide formulation with the addition of fluoride offers a significant increase in protection against a cariogenic acid challenge compared to a fluoride solution.

**Table 5. Relative microhardness values for human enamel exposed to a cariogenic lactic acid buffer, following treatment with nano zinc oxide suspension, nano zinc oxide suspension with fluoride solution, fluoride solution and water.**

| | **VHN compared to baseline** | | |
|---|---|---|---|
| **Test Agent** | **Baseline** | **After treatment / acid challenge** | **SD** |
| 0.50% ZnO | 100 | 71 | 2 |
| 0.50% ZnO +300 ppm F | 100 | 84 | 3 |
| 300 ppm F | 100 | 70 | 3 |
| Water | 100 | 68 | 3 |

## Claims

1. An oral care composition comprising nanoparticulate zinc oxide and a dispersing agent in the absence of palatinit for use in a method of helping to prevent, inhibit and/or treat dental erosion.

2. A composition according claim 1 wherein the zinc oxide is present in an amount of 0.001 to 10.0% by weight of the total composition.

3. A composition according to any one of claims 1 to 2 comprising a source of fluoride ions.

4. A composition according to claim 3 wherein the fluoride ion source is an alkali metal fluoride, an alkali metal monofluorophosphate, stannous fluoride, or an amine fluoride.

5. A composition according to any one of claims 1 to 4 comprising a desensitising agent.

6. A composition according to claim 5 wherein the desensitising agent is a strontium salt or a potassium salt.

7. A dentifrice composition according to claim 1 comprising particulate zinc oxide, a fluoride ion source and a silica dental abrasive, the dentifrice having a Relative Dentine Abrasivity (RDA) value from 20 to 60 and a pH in the range 5.5 to 9.0 and being free of an orthophosphate buffer or a water-soluble salt of a C₁₀₋₁₈ alkyl sulphate.

8. A dentifrice composition according to claim 1 comprising particulate zinc oxide, a fluoride ion source, a thickening system comprising a thickening silica in combination with xanthan gum optionally with carrageenan and/or a carbomer, an anionic surfactant, and a silica dental abrasive in an amount up to 20% by weight of the total composition, the dentifrice having a pH in the range from 5.5 to 9.0, and being free of an orthophosphate buffer or a calcium salt.

## Patentansprüche

1. Mundpflegezusammensetzung, umfassend nanopartikuläres Zinkoxid und ein Dispergiermittel in Abwesenheit von Palatinit zur Verwendung in einem Verfahren, das dabei hilft, Zahnerosion zu verhindern, zu inhibieren und/oder zu behandeln.

2. Zusammensetzung gemäß Anspruch 1, wobei das Zinkoxid in einer Menge von 0,001 bis 10,0 Gew.% der Gesamtzusammensetzung vorhanden ist.

3. Zusammensetzung gemäß mindestens einem der Ansprüche 1 bis 2, die eine Fluoridionenquelle umfasst.

4. Zusammensetzung gemäß Anspruch 3, wobei die Fluoridionenquelle ein Alkalimetallfluorid, ein Alkalimetallmonofluorphosphat, Zinnfluorid oder ein Aminfluorid ist.

5. Zusammensetzung gemäß mindestens einem der Ansprüche 1 bis 4, die ein Desensibilisierungsmittel umfasst.

6. Zusammensetzung gemäß Anspruch 5, wobei das Desensibilisierungsmittel ein Strontiumsalz oder ein Kaliumsalz ist.

7. Zahnpflegemittelzusammensetzung gemäß Anspruch 1, umfassend partikuläres Zinkoxid, eine Fluoridionenquelle und ein Silica-Zahnschleifmittel, wobei das Zahnpflegemittel einen relativen Dentin-Abrasivitäts-(RDA)-Wert von 20 bis 60 und einen pH-Wert im Bereich von 5,5 bis 9,0 aufweist und frei von einem Orthophosphatpuffer oder einem wasserlöslichen Salz eines C₁₀₋₁₈-Alkylsulfats ist.

8. Zahnpflegemittezusammensetzung gemäß Anspruch 1, umfassend partikuläres Zinkoxid, eine Fluoridionenquelle, ein Verdickungssystem, das ein Silica-Verdickungsmittel in Kombination mit Xanthangummi gegebenenfalls mit Carrageenan und/oder einem Carbomer umfasst, ein anionisches Tensid und ein Silica-Zahnschleifmittel in einer Menge von bis zu 20 Gew.% der Gesamtzusammensetzung, wobei das Zahnpflegemittel einen pH-Wert im Bereich von 5,5 bis 9,0 aufweist und frei von einem Orthophosphatpuffer oder einem Calciumsalz ist.

## Revendications

1. Composition de soin buccal comprenant des nanoparticules d'oxyde de zinc et un agent de dispersion en l'absence de palatinit pour une utilisation dans un procédé d'aide à la prévention, à l'inhibition et/ou au traitement de l'érosion dentaire.

2. Composition selon la revendication 1 dans laquelle l'oxyde de zinc est présent en une quantité de 0,001 à 10,0 % en poids de la composition totale.

3. Composition selon la revendication 1 ou 2 comprenant une source d'ions fluorure.

4. Composition selon la revendication 3 dans laquelle la source d'ions fluorure est un fluorure de métal alcalin, un monofluorophosphate de métal alcalin, du fluorure stanneux ou un fluorure d'aminé.

5. Composition selon l'une quelconque des revendications 1 à 4 comprenant un agent désensibilisant.

6. Composition selon la revendication 5 dans laquelle l'agent désensibilisant est un sel de strontium ou un sel de potassium.

7. Composition de dentifrice selon la revendication 1 comprenant des particules d'oxyde de zinc, une source d'ions fluorure et un abrasif dentaire à base de silice, le dentifrice présentant une valeur d'abrasivité relative de la dentine (RDA) de 20 à 60 et un pH de 5,5 à 9,0 et ne contenant pas de tampon à base d'orthophosphate ou de sel hydrosoluble de sulfate d'alkyle en C₁₀₋₁₈.

8. Composition de dentifrice selon la revendication 1 comprenant des particules d'oxyde de zinc, une source d'ions fluorure, un système épaississant comprenant une silice épaississante en combinaison avec de la gomme xanthane, facultativement avec du carraghénane et/ou un carbomère, un tensioactif anionique et un abrasif dentaire à base de silice en une quantité allant jusqu'à 20 % en poids de la composition totale, le dentifrice ayant un pH dans la plage de 5,5 à 9,0 et ne contenant pas de tampon à base d'orthophosphate ou de sel de calcium.
